# EUROPÄISCHE PATENTSCHRIFT

(11) **EP 0 675 975 B1**
(45) Veröffentlichungstag und Bekanntmachung des Hinweises auf die Patenterteilung: **16.10.1996**
(21) Anmeldenummer: 94901946.7
(22) Anmeldetag: 02.12.1993
(51) Int. Cl.: C25D 3/12, C07C 335/32

(54) **VERWENDUNG VON THIURONIUMSALZEN ALS GLANZMITTEL FÜR WÄSSRIG-SAURE GALVANISCHE NICKELBÄDER**
USE OF THIOUREA SALTS AS BRIGHTENERS FOR AQUEOUS-ACID GALVANIC NICKEL BATHS
UTILISATION DE SELS DE THIO-UREE COMME BRILLANTEURS POUR BAINS GALVANIQUES ACIDES-AQUEUX AU NICKEL

(30) Priorität: 15.12.1992 DE 4242194
(43) Veröffentlichungstag der Anmeldung: 11.10.1995
(73) Patentinhaber: BASF Aktiengesellschaft, 67063 Ludwigshafen (DE)
(72) Erfinder: BURKHART, Bernd, D-67112 Mutterstadt (DE); OFTRING, Alfred, D-67098 Bad Duerkheim (DE); SCHWENDEMANN, Volker, D-67434 Neustadt (DE); SCHROEDER, Ulrich, D-67227 Frankenthal (DE); GLASER, Klaus, D-68161 Mannheim (DE)
(86) Internationale Anmeldenummer: EP9303383
(87) Internationale Veröffentlichungsnummer: WO9413862

(56) Entgegenhaltungen:
- EP-A- 0 193 303
- DD-A- 250 161
- US-A- 3 882 009
- PROTECTION OF METALS Bd. 27, Nr. 2 , März 1991 , US Seiten 258 - 261 XP261670 MILUSHKIN 'using new thiourea derivatives as brighteners in nickel plating'

## Beschreibung

Die vorliegende Erfindung betrifft ein Verfahren zur Herstellung vernickelter Formteile durch galvanisches Abscheiden von Nickel aus wäßrig-sauren Bädern unter Verwendung von Thiuroniumsalzen als Glanzmittel. Weiterhin betrifft die Erfindung einen Teil der Thiuroniumsalze selbst, insofern diese neue Stoffe darstellen.

Es ist bekannt, daß saure Nickelelektrolyte in geringer Menge organische Substanzen enthalten müssen, wenn bei der galvanischen Nickelabscheidung eine glänzende, duktile und an der Oberfläche ebene Abscheidung des Metalls erzielt werden soll. Derartige Glanzmittel, die man in der Regel in primäre Glanzmittel ("Glanzträger") und sekundäre Glanzmittel ("Glanzbildner") unterteilt, werden üblicherweise als Kombination aus mehreren dieser Mittel eingesetzt, um die Wirkung zu steigern.

In der Literaturstelle "Praktische Galvanotechnik", Eugen G. Leuze Verlag, Saulgau, 4. Auflage 1984, Seite 268 bis 271 (1) werden Substanzklassen für übliche Glanzmittel für Nickelelektrolyte beschrieben. Eine Einteilung in primäre und sekundäre Glanzmittel und Einebner wird zwar getroffen, es wird aber gleichzeitig darauf hingewiesen, daß diese Einteilung nicht immer eindeutig vorgenommen werden kann. Als glanzerzeugende Substanzklassen werden genannt:
- Sulfonimide, z.B. Benzoesäuresulfimid
- Sulfonamide
- Benzolsulfonsäuren, z.B. Mono-, Di- und Tribenzolsulfonsäure
- Naphthalinsulfonsäuren, z.B. Mono,- Di- und Trinaphthalinsulfonsäure
- Alkylsulfonsäuren
- Sulfinsäure
- Arylsulfonsulfonate
- aliphatische Verbindungen mit Ethylen- und/oder Acetylenbindungen, z.B. Butindiol
- ein- und mehrkernige stickstoffhaltige Heterocyclen, welche noch weitere Heteroatome wie Schwefel oder Selen enthalten können
- Cumarin
- Amine und quaternäre Ammoniumverbindungen als Einebnungsmittel
- Saccharin.

In der DE-B 11 91 652 (2) werden ein- oder mehrkernige heterocyclische Stickstoffbasen vom aromatischen Typ in quaternisierter Form wie Pyridiniumsalze, z.B. 2-Pyridinium-1-sulfatoethan, als Einebnungsmittel, d.h. Glanzmittel, für saure galvanische Nickelbäder beschrieben. Diese Mittel werden zuammen mit üblichen Grundglanzmitteln wie Benzol-m-disulfonsäure, Diaryldisulfimiden oder Sulfonamiden verwendet.

Aus der US-A 3 630 857 (3) ist bekannt, Nitrilgruppen enthaltende Thioharnstoffderivate der allgemeinen Formel 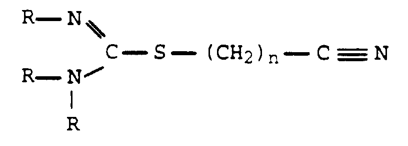 in der die Variablen R unabhängig voneinander Wasserstoff, Alkyl, Alkenyl oder Alkinyl mit jeweils 1 bis 4 C-Atomen bedeuten und n für eine Zahl von 1 bis 4 steht, als Glanzmittel für wäßrig-saure galvanische Nickelbader einzusetzen.

In der Praxis werden üblicherweise als Glanzbildner Alkenylsulfonsäuren wie Natriumvinylsulfonat oder Natriumallylsulfonat in Kombination mit anderen Glanzbildnern wie Propargylalkohol, 2-Butin-1,4-diol, Propinsulfonsäure oder 3-Pyridinium-propylsulfonat verwendet.

Die Schrift Protection of Metals, Bd. 27, Nr.2 (1991), US, 5.258-61, Milushkin: "Using New Thiourea Derivatives as Brighteners in Nickel Plating" schlägt Thiuronium-Salze als Glanzbildner für saure Nickelbäder vor, die sich von denen der Formel I der vorliegenden Anmeldung durch den Substituenten in β-Stellung zum Schwefelatom unterscheiden.

Nachteilig bei den aus dem Stand der Technik bekannten Mitteln ist allerdings die in der Regel relativ hohe Einsatzkonzentration in den verwendeten Nickelelektrolyt-Bädern.

Der Erfindung lag daher ein verbessertes Verfahren zur Herstellung vernickelter Formteile unter Verwendung von Glanzmitteln, die bei besserer oder zumindest gleicher Glanzbildung wie bei beispielsweise 2-Pyridinium-1-sulfatoethan oder 3-Pyridiniumpropylsulfonat in geringerer Konzentration eingesetzt zu werden brauchen, als Aufgabe zugrunde.

Demgemäß wurde ein Verfahren zur Herstellung vernickelter Formteile durch galvanisches Abscheiden von Nickel aus wäßrig-sauren Bädern, die als wesentliche Bestandteile ein oder mehrere Nickelsalze, eine oder mehrere anorganische Säuren und ein oder mehrere Glanzmittel enthalten, gefunden, welches dadurch gekennzeichnet ist, daß man als Glanzmittel Thiuroniumsalze der allgemeinen Formel I 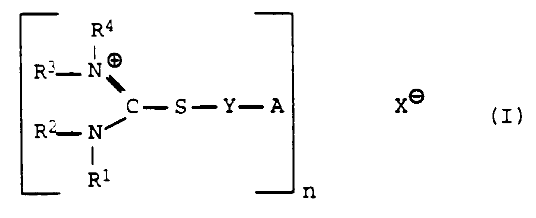 in der
- R¹ bis R⁴: Wasserstoff, C₁- bis C₁₈-Alkyl, welches durch Carboxylgruppen, C₁- bis C₄-Alkoxycarbonylgruppen oder Cyanogruppen substituiert sein kann, C₂- bis C₁₂-Alkenyl, C₂- bis C₁₂-Alkinyl, C₅ bis C₈-Cycloalkyl, C₇- bis C₁₂-Phenylalkyl oder Phenyl, welches durch ein oder zwei C₁- bis C₄-Alkylreste, C₁- bis C₄-Alkoxyreste, Halogenatome, Hydroxylgruppen, Phenylreste oder C₁- bis C₄-Alkoxycarbonylgruppen substituiert sein kann, bedeuten,
- Y: eine chemische Bindung oder lineares oder verzweigtes Alkylen, Alkenylen oder Alkinylen mit jeweils bis zu 20 C-Atomen bezeichnet,
- A: Wasserstoff oder eine Gruppierung der Formel -CO-H, -CO-R⁵, -CO-OH, -CO-OR⁵, -CO-NR⁶R⁷, -CO-CH₂-CO-OR⁵, -O-CO-H, -O-CO-R⁵, -NR⁶⁻CO-R⁵, -OR⁵, -SO₂-R⁵, -SO₂-OH, -SO₂-OR⁵, -PO(OH)₂, -PO(OH)(OR⁵), -PO(OR⁵)₂, OPO(OH)₂, -OPO(OH) (OR⁵) oder -OPO(OR⁵)₂ bezeichnet, wobei
- R⁵: C₁- bis C₁₂-Alkyl, C₂- bis C₁₂-Alkenyl, C₂- bis C₁₂-Alkinyl, C₅- bis C₈-Cycloalkyl, C₇- bis C₁₂-Phenylalkyl oder Phenyl, welches durch ein oder zwei C₁- bis C₄-Alkylreste, Halogenatome, Hydroxylgruppen, Phenylreste oder C₁- bis C₄-Alkoxycarbonylgruppen substituiert sein kann, bedeutet und
- R⁶ und R⁷: Wasserstoff oder C₁- bis C₄-Alkyl bedeuten,
- n: für eine Zahl von 1 bis 4 steht und
- X^{⊖}: ein n-wertiges anorganisches oder organisches Anion, welches die Wasserlöslichkeit fördert, bezeichnet,
einsetzt.

Als C₁- bis C₄-Alkylreste für R⁶ bis R¹⁰, R¹², R¹³ und am Phenylkern kommen n-Propyl, iso-Propyl, n-Butyl, iso-Butyl, sec.-Butyl, tert.-Butyl und vor allem Methyl und Ethyl in Betracht.

Als geradkettige oder verzweigte C₁- bis C₁₈- bzw. C₁- bis C₁₂-Alkylreste für R¹ bis R⁴, R⁵ und R¹¹ kommen beispielsweise neben den oben aufgeführten C₁- bis C₄-Alkylresten n-Amyl, iso-Amyl, sek.-Amyl, tert.-Amyl, Neopentyl, n-Hexyl, n-Heptyl, n-Octyl, 2-Ethylhexyl, n-Nonyl, iso-Nonyl, n-Decyl, n-Undecyl, n-Dodecyl, n-Tetradecyl, n-Hexadecyl und n-Octadecyl in Betracht. Bevorzugt werden hiervon C₁- bis C₄-Alkylreste.

Als durch Carboxylgruppen, C₁- bis C₄-Alkoxycarbonylgruppen oder Cyanogruppen substituiertes C₁- bis C₁₈-Alkyl für R¹ bis R⁴ eignet sich beispielsweise 2-Carboxyethyl, 2-(Methoxycarbonyl)ethyl, 2-(Ethoxycarbonyl)ethyl oder 2-Cyanoethyl.

Als C₅- bis C₈-Cycloalkylreste für R¹ bis R⁴, R⁵ und R¹¹ kommen insbesondere Cyclopentyl, Cyclohexyl, Cycloheptyl, Cyclooctyl, Methylcyclopentyl, Dimethylcyclopentyl, Methylcyclohexyl, Dimethylcyclohexyl und Ethylcyclohexyl in Betracht. Bevorzugt werden hiervon Cyclopentyl und Cyclohexyl.

Als C₇- bis C₁₂-Phenylalkylgruppen für R¹ bis R⁴, R⁵ und R¹¹ eignen sich beispielsweise 1-Phenylethyl, 2-Phenylethyl, l-Phenylpropyl, 2-Phenylpropyl, 3-Phenylpropyl, 2-Phenylprop-2-yl, 4-Phenylbutyl, 2,2-Dimethyl-2-phenylethyl, 5-Phenylamyl, 6-Phenylhexyl und vor allem Benzyl.

Bei Verwendung monosubstituierter Phenylreste für R¹ bis R⁴, R⁵ und R¹¹ ist das Substitutionsschema ortho, meta oder vorzugsweise para, bei disubstituierten Phenylresten stehen die Substituenten vor allem in 2,4-Position, z.B. bei 2,4-Xylyl. Es wird bei Vorhandensein von Substituenten ein Substitionsgrad von 1 bevorzugt. Besonders bevorzugt wird jedoch unsubstituiertes Phenyl.

Als C₁- bis C₄-Alkoxyreste kommen insbesondere Methoxy und Ethoxy, daneben aber auch n-Propoxy, n-Butoxy, iso-Butoxy, sec.-Butoxy und tert.-Butoxy in Betracht.

Als C₁- bis C₄-Alkoxycarbonylgruppen dienen beispielsweise n-Propoxycarbonyl, iso-Propoxycarbonyl, n-Butoxycarbonyl, iso-Butoxycarbonyl, sec.-Butoxycarbonyl, tert.-Butoxycarbonyl, vor allem aber Ethoxycarbonyl und Methoxycarbonyl.

Der Begriff Halogenatom umfaßt hierbei Fluor, Jod, vor allem aber Brom und insbesondere Chlor.

Als geradkettige oder verzweigte C₂- bis C₁₂-Alkylenreste für R¹ bis R⁴, R⁵ und R¹¹ eignen sich z.B. Vinyl, Allyl, Methallyl, 3-Butenyl, 4-Pentenyl, 5-Hexenyl, 7-Octenyl, 9-Decenyl, 11-Dodecenyl, Citronellolyl, Geraniolyl oder Linaloolyl.

Als geradkettige oder verzweigte C₂- bis C₁₂-Alkinylreste für R¹ bis R⁴, R⁵ und R¹¹ eignen sich z.B. Ethinyl oder 2-Propinyl.

Die Reste R⁶ bis R¹⁰, R¹² und R¹³ stehen vorzugsweise für Wasserstoff, Methyl oder Ethyl.

m steht vorzugsweise für eine Zahl von 0 bis 8, insbesondere 0 bis 5, ganz besonders 0 bis 3.

Als n-wertige Anionen X kommen die üblichen, normalerweise die Wasserlöslichkeit fördernden anorganischen oder organischen Anionen in Betracht, so vor allem Chlorid, Bromid, Fluorid, Sulfat, Hydrogensulfat, Methansulfonat, Trifluormethansulfonat, 2-Hydroxyethansulfonat, p-Toluolsulfonat, Nitrat, Tetrafluorborat, Perchlorat, l-Hydroxyethan-1,1-diphosphonat, Dihydrogenphosphat, Hydrogenphosphat, Phosphat, Formiat, Acetat, Oxalat, Citrat und Tartrat.

Hiervon werden ein- oder zweifach (n=1 oder 2) geladene Anionen, vor allem Fluorid, Sulfat, Methansulfonat, Nitrat und Tetrafluorborat, insbesondere jedoch Chlorid und Bromid bevorzugt.

Bedeutet die Variable A eine Carbonsäure-, Sulfonsäure-, Phosphonsäure- oder Phosphorsäure-Funktion, können die Thiuroniumsalze I auch als Betaine vorliegen, die durch Abspaltung von HX aus den Thiuroniumsalzen I entstehen können.

In einer bevorzugten Ausführungsform werden Thiuroniumsalze I eingesetzt, bei denen die Variablen R¹ bis R⁴ Wasserstoff, C₁- bis C₄-Alkyl, Allyl oder Phenyl, welches durch ein oder zwei C₁- bis C₄-Alkylreste substituiert sein kann, bedeuten. Insbesondere bedeuten alle Variablen R¹ bis R⁴ Wasserstoff oder drei Wasserstoff und eine Allyl.

In einer weiteren bevorzugten Ausführungsform werden Thiuroniumsalze der allgemeinen Formel Ia 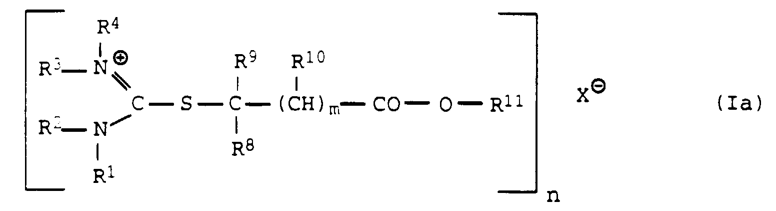 eingesetzt, in der die Variablen R¹ bis R⁴, n und X^{⊖} die oben genannten Bedeutungen haben, R⁸ bis R¹⁰ Wasserstoff oder C₁- bis C₄-Alkyl bezeichnen, R¹¹ Wasserstoff, C₁- bis C₁₂-Alkyl, C₂- bis C₁₂-Alkenyl, C₂- bis C₁₂-Alkinyl, C₅- bis C₈-Cycloalkyl, C₇- bis C₁₂-Phenylalkyl oder Phenyl, welches durch ein oder zwei C₁- bis C₄-Alkylreste substituiert sein kann, bedeutet und m für eine Zahl von 0 bis 10 steht.

In einer weiteren bevorzugten Ausführungsform werden Thiuroniumsalze der allgemeinen Formel Ib 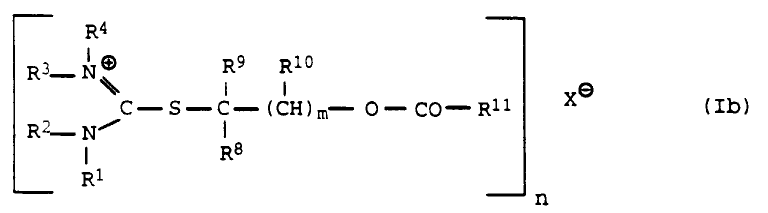 eingesetzt, in der die Variablen R¹ bis R⁴, R⁸ bis R¹¹, m, n und X^{⊖} die oben genannten Bedeutungen haben.

In einer weiteren bevorzugten Ausführungsform werden Thiuroniumsalze der allgemeinen Formel Ic 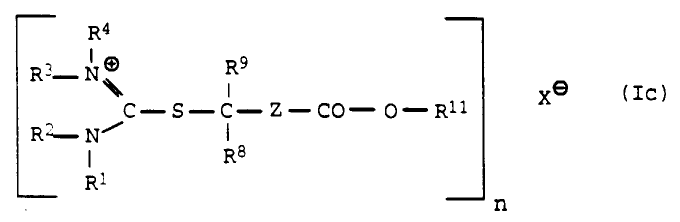 eingesetzt, in der die Variable R¹ bis R⁴, R⁸, R⁹, R¹¹, n und X^{⊖} die oben genannten Bedeutungen haben und Z für eine Gruppierung der Formel -CR¹²=CR¹³ - oder -C≡C- steht, wobei R¹² und R¹³ Wasserstoff oder C₁- bis C₄-Alkyl bezeichnen.

In einer weiteren bevorzugten Ausführungsform werden Thiuroniumsalze der allgemeinen Formel Id 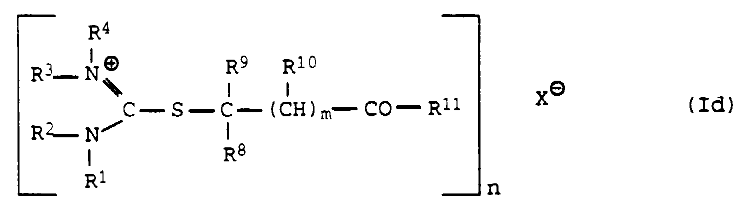 eingesetzt, in der die Variablen R¹ bis R⁴, R⁸ bis R¹¹, m, n und X^{⊖} die oben genannten Bedeutungen haben.

In einer weiteren bevorzugten Ausführungsform werden Thiuroniumsalze der allgemeinen Formel Ie 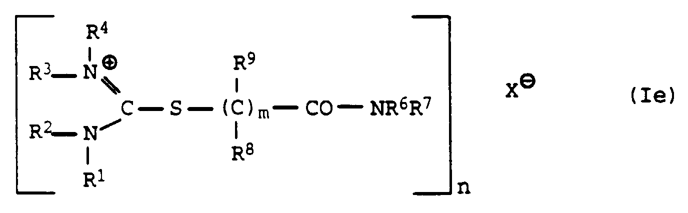 eingesetzt, in der die Variablen R¹ bis R⁴, R⁶ bis R⁹, m, n und X^{⊖} die oben genannten Bedeutungen haben.

In einer weiteren bevorzugten Ausführungsform werden Thiuroniumsalze der allgemeinen Formel If 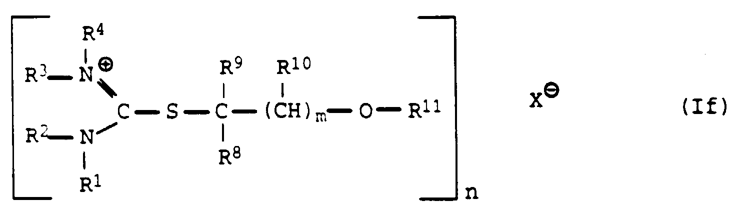 eingesetzt, in der die Variablen R¹ bis R⁴, R⁸ bis R¹¹, m, n und X^{⊖} die oben genannten Bedeutungen haben.

In einer weiteren bevorzugten Ausführungsform werden Thiuroniumsalze der allgemeinen Formel Ig 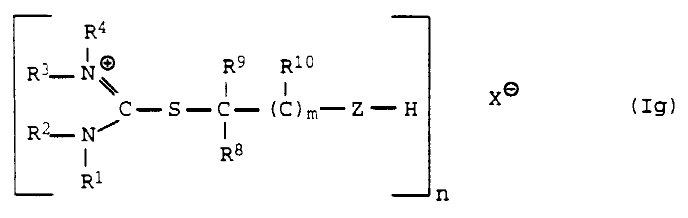 eingesetzt, in der die Variablen R¹ bis R⁴, R⁸, R⁹, Z, m, n und X^{⊖} die oben genannten Bedeutungen haben.

Da die Thiuroniumsalze I normalerweise den Wirkungscharakter von sekundären Glanzmitteln haben, werden sie vorzugsweise in Kombination mit mindestens einem weiteren, normalerweise primären Glanzmittel, gegebenenfalls auch mit einem oder mehreren weiteren sekundären Glanzmitteln verwendet. Als primäre Glanzmittel kommen beispielsweise Vinylsulfonsäure- oder Allylsulfonsäure-Natriumsalz, als sekundäre Glanzmittel beispielsweise 2-Butin-1,4-diol oder Propargylalkohol in Frage.

Die verwendeten wäßrig-sauren Nickelelktrolyt-Bäder enthalten ein oder meist mehrere Nickelsalze, z.B. Nickelsulfat und Nickelchlorid, eine oder mehrere anorganische Säuren, vorzugsweise Borsäure und Schwefelsäure, als Glanzmittel die Verbindungen I allein oder vorzugsweise in Kombination mit weiteren üblichen Glanzmitteln sowie gegebenenfalls weitere übliche Hilfsmittel und Zusätze in den hierfür üblichen Konzentrationen, z.B. Netzmittel und Porenverhütungsmittel. Gebräuchlich waßrig-saure Nickelelektrolyte ("Watts-Elektrolyte") haben die folgende Grundzusammensetzung:
200 bis 350 g/l NiSO₄.7 H₂O
30 bis 150 g/l NiCl₂.6 H₂O
30 bis 50 g/l H₃BO₃.

Der pH-Wert der Elektrolyt-Bäder liegt üblicherweise zwischen 3 und 6, vorzugsweise zwischen 4 und 5. Zur Einstellung dieses pH-Wertes dient zweckmäßigerweise eine starke Mineralsäure, vorzugsweise Schwefelsäure.

Die Verbindungen I liegen in den Elektrolyt-Bädern in niedrigen Konzentrationen, in der Regel zwischen 0,01 und 0,5 g/l, vorzugsweise zwischen 0,025 und 0,3 g/l, vor. Die Konzentrationen weiterer üblicher Glanzmittel liegen normalerweise im Bereich von jeweils 0,1 bis 10 g/l, insbesondere 0,1 bis 2,0 g/l.

Mit den beschriebenen Nickelelektrolyt-Bädern können vor allem Nickelüberzüge auf Formteilen aus Stahl, daneben aber auch auf Formteilen aus anderen Materialien, beispielsweise Messing, die wie üblich vorbehandelt worden sind, galvanisch erzeugt werden. Hierzu arbeitet man in der Regel bei Temperaturen von 30°C bis 80°C, vorzugsweise 40 bis 60°C.

Die erfindungsgemäß verwendeten Verbindungen I zeichnen sich durch eine außerordentlich hohe Glanzbildung aus. Man erreicht mit ihnen in der Regel einen stärkeren Glanz als mit den üblichen Glanzbildnern und das meist bei deutlich niedrigerer Dosierung in den Nickelelektrolyt-Badern.

Die beschriebenen Thiuroniumsalze I lassen sich in vorteilhafter Weise durch Umsetzung der entsprechenden Vorstufe der allgemeinen Formel II

Nuc-Y-A (II)

in der Nuc eine nucleofuge Abgangsgruppe, vorzugsweise Chlor oder Brom, darstellt, mit einem Thioharnstoff der allgemeinen Formel III 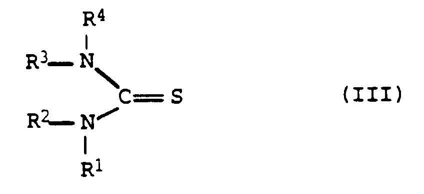 und gewünschtenfalls anschließenden Austausch des Anions Nuc^{⊖} gegen X^{⊖} herstellen.

Als Thioharnstoffe III eignen sich beispielsweise unsubstituierter Thioharnstoff, N-Methylthioharnstoff, N,N'-Dimethylthioharnstoff, N,N,N',N'-Tetramethylthioharnstoff, N-Ethylthioharnstoff, N,N'-Diethylthioharnstoff, N,N,N',N'-Tetraethylthioharnstoff, N-Phenylthioharnstoff, N,N'-Diphenylthioharnstoff, N-PhenylN-methylthioharnstoff, N-Phenyl, N'-methylthioharnstoff, N,N'-Di-butylthioharnstoff, N-Benzylthioharnstoff, N-Allylthioharnstoff oder N,N'-Dicyclohexylthioharnstoff.

Die Umsetzung der Komponenten II und III wird zweckmäßigerweise in einem inerten organischen Lösungsmittel wie Toluol, Xylol, Petrolether, Ligroin, Cyclohexan, Aceton, Tetrahydrofuran, Dioxan, Methanol, Ethanol, iso-Propanol, Essigsäureethylester oder Benzoesäuremethylester oder in einer Mischung hieraus durchgeführt. Man kann die Umsetzung aber auch in Wasser oder in einem einphasigen oder zweiphasigen Gemisch aus Wasser und einem oder mehreren organischen Lösungsmitteln, vorzugsweise polaren organischen Lösungsmitteln, vornehmen. Bei Zweiphasengemischen kann ein üblicher Phasentransferkatalysator eingesetzt werden. Man arbeitet in der Regel bei Temperaturen von 40 bis 130°C, insbesondere bei 60 bis 110°C, und bei Normaldruck.

Da ein Teil der beschriebenen Thiuroniumsalze I neue Stoffe darstellt, betrifft die vorliegende Erfindung weiterhin diese neuen Verbindungen.

Demgemäß sind Gegenstand der vorliegenden Erfindung Thiuroniumsalze der allgemeinen Formel Ih 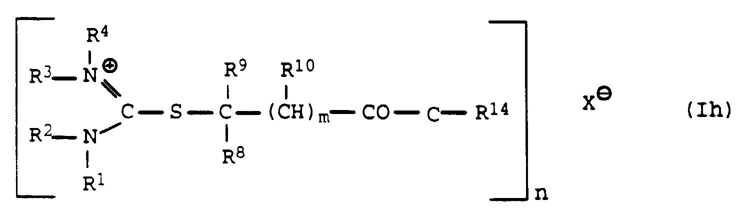 in der die Variablen R¹ bis R⁴, R⁸ bis R¹⁰, m, n und X^{⊖} die oben genannten Bedeutungen haben und R¹⁴ für C ₇- bis C₁₂-Phenylalkyl oder Phenyl, welches durch ein oder zwei C₁- bis C₄-Alkylreste substituiert sein kann, steht.

Weiterhin sind Gegenstand der vorliegenden Erfindung Thiuroniumsalze der allgemeinen Formel Ij 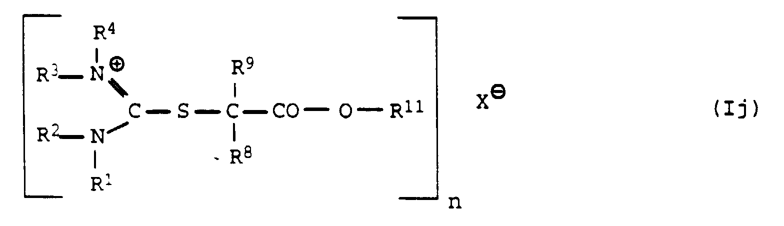 in der die Variablen R¹ bis R⁴, R⁸, R⁹, R¹¹, n und X^{⊖} die oben genannten Bedeutungen haben mit der Maßgabe, daß mindestens einer der Reste R⁸ und R⁹ von Wasserstoff verschieden ist.

Weiterhin sind Gegenstand der vorliegenden Erfindung Thiuroniumsalze der allgemeinen Formel Ib 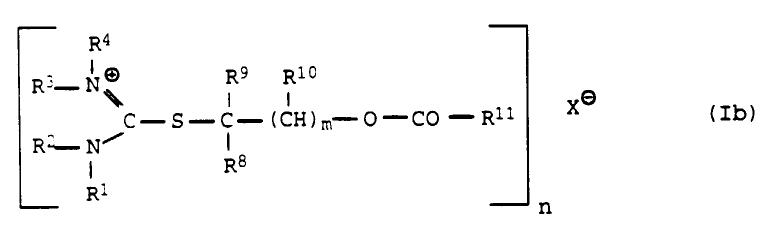 in der die Variablen R¹ bis R⁴, R⁸ bis R¹¹, m, n und X^{⊖} die oben genannten Bedeutungen haben.

Weiterhin sind Gegenstand der vorliegenden Erfindung Thiuroniumsalze der allgemeinen Formel Ik 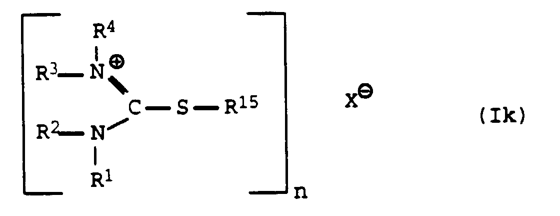 in der die Variablen R¹ bis R⁴, n und X^{⊖} die oben genannten Bedeutungen haben und R¹⁵ für die Gruppierung 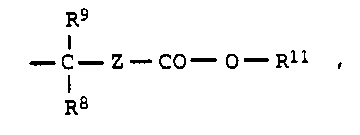 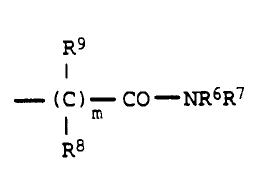 oder 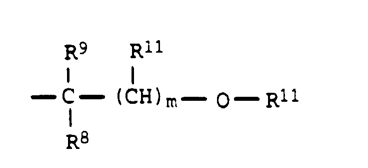 steht, wobei die Variablen R⁶ bis R¹¹, Z und m die oben genannten Bedeutungen haben, mit der Maßgabe, daß mindestens einer der Reste R¹ bis R⁴ eine Allylgruppe bezeichnet.

Die Thiuroniumsalze Ih und Ij stellen Untergruppen der Verbindungen Ia und die Thiuroniumsalze Ik Untergruppen der Verbindungen Ic, Ie und If dar. Gleichzeitig ist die Verwendung der Thiuroniumsalze Ih, Ij und Ik im erfindungsgemäßen Verfahren zur Herstellung vernickelter Formteile jeweils als weitere bevorzugte Ausführungsform anzusehen.

### Herstellungsbeispiele

### Beispiel 1

### Herstellung von Thiuroniumessigsäurebenzylester-chlorid

7,6 g (0,1 mol) Thioharnstoff wurden in 50 ml Aceton gelöst. Dazu tropfte man 18,5 g (0,1 mol) Chloressigsäurebenzylester und erhitzte zwei Stunden zum Sieden. Nach Abkühlen auf Raumtemperatur wurde das ausgefallene Produkt abfiltriert und mit Diethylether gewaschen. Nach Trocknen erhielt man 23,5 g (entsprechend einer Ausbeute von 90 %) der Titelverbindung in Form von farblosen Kristallen. Die Reinheit des Produktes lag bei über 99 %.

### Beispiel 2

### Herstellung von Thiuronium-2-ethylessigsäure-bromid

Die Titelverbindung wurde analog zu Beispiel 1 aus Thioharnstoff und 2-Brombuttersäure in einer Ausbeute von 79 % hergestellt.

### Beispiel 3

### Herstellung von Thiuronium-2,2-dimethylessigsäure-chlorid

Die Titelverbindung wurde analog zu Beispiel 1 aus Thioharnstoff und 2-Chlor-2-methylpropionsäure in einer Ausbeute von 74 % hergestellt.

### Beispiel 4

### Herstellung von Thiuronium-N,N-dimethylessigsäureamid-chlorid

Die Titelverbindung wurde analog zu Beispiel 1 aus Thioharnstoff und 2-Chlor-N,N-dimethylacetamid in einer Ausbeute von 89 % hergestellt.

### Beispiel 5

### Herstellung von 3-Thiuroniunpropyl-methylketon-chlorid

Die Titelverbindung wurde analog zu Beispiel 1 aus Thioharnstoff und 3-Chlorpropyl-methylketon in einer Ausbeute von 71 % hergestellt.

### Beispiel 6

### Herstellung von N'-Allylthiuronium-N,N-dimethylessigsäureamidchlorid

Die Titelverbindung wurde analog zu Beispiel 1 aus N-Allylthioharnstoff und 2-Chlor-N,N-dimethylacetamid in einer Ausbeute von 79 % hergestellt.

### Beispiel 7

### Herstellung von 2-(N-Allylthiuronium)ethyl-methylether-bromid

Die Titelverbindung wurde analog zu Beispiel 1 aus N-Allylthioharnstoff und 2-Bromethyl-methylether in einer Ausbeute von 69 % hergestellt.

### Beispiel 8

### Herstellung von 4- (N-Allylthiuronium)-2-methylcrotonsaure-bromid

Die Titelverbindung wurde analog zu Beispiel 1 aus N-Allylthioharnstoff und 4-Brom-2-methylcrotonsäure in einer Ausbeute von 70 % hergestellt.

### Anwendungsbeispiele

Die gemäß den Beispielen 1 bis 8 hergestellten Produkte wurden als Glanzmittel in schwach sauren galvanischen Bädern zur Abscheidung von Nickel eingesetzt.

Der verwendete wäßrige Nickelelektrolyt hatte folgende Zusammensetzung:
- 300 g/l: NiSO₄ . 7 H₂O
- 60 g/l: NiCl₂ . 6 H₂O
- 45 g/l: H₃BO₃
- 2 g/l: Saccharin
- 0,8 g/l: Vinylsulfonsäure-Natriumsalz
- x g/l: Glanzmittel gemäß Tabelle
- 0,5 g/l: eines Fettalkoholderivates der Formel C₁₂H₂₅/C₁₄H₂₉-O-(CH₂CH₂O)₂-SO₃Na als Netzmittel

Der pH-Wert des Elektrolyten wurde mit Schwefelsäure auf 4,2 eingestellt.

Es wurden Messingbleche verwendet, die vor der Beschichtung mit Nickel in einem alkalischen Elektrolyten nach den üblichen Methoden kathodisch entfettet wurden. Die Nickelabscheidung erfolgte in einer 250 ml-Hull-Zelle bei 55°C und einer Stromstärke von 2A während eines Zeitraums von 10 Minuten. Anschließend wurden die Bleche mit Wasser gespült und mit Preßluft getrocknet.

Die folgende Tabelle zeigt die Ergebnisse dieser Versuche. Man erkennt, daß mit den erfindungsgemäßen Glanzmitteln ein stärkerer Glanz erzielt wurde als mit den Glanzmitteln des Standes der Technik, und das zum Teil bei deutlich niedrigerer Dosierung in den Nickelelektrolyt-Bädern.

**Tabelle**

| Prüfergebnisse der galvanischen Nickelabscheidung | | | |
|---|---|---|---|
| Beispiel Nr. | Glanzmittel | Konzentration | Glanz |
| 9 | aus Beispiel Nr. 1 | 0,1 | 5 |
| 10 | aus Beispiel Nr. 2 | 0,2 | 5 |
| 11 | aus Beispiel Nr. 3 | 0,2 | 5 |
| 12 | aus Beispiel Nr. 4 | 0,2 | 5 |
| 13 | aus Beispiel Nr. 5 | 0,2 | 5 |
| 14 | aus Beispiel Nr. 6 | 0,2 | 5 |
| 15 | aus Beispiel Nr. 7 | 0,2 | 5 |
| 16 | aus Beispiel Nr. 8 | 0,3 | 5 |
| zum Vergleich | | | |
| A | 2-Pyridinium-1-sulfatoethan | 0,3 | 4 - 5 |
| B | 3-Pyridinium-propylsulfonat | 0,3 | 4 - 5 |

Benotungsschema für den Glanz:
5 = hervorragend (perfekter Spiegelglanz)
4 = gut (nahezu Spiegelglanz)
3 = mäßig
2 = schlecht
1 = kein Glanz

Die Vergleichsverbindung A ist aus (2) bekannt.
Konzentration: [g/l]

## Patentansprüche

1. Verfahren zur Herstellung vernickelter Formteile durch galvanisches Abscheiden von Nickel aus wäßrig-sauren Bädern, die als wesentliche Bestandteile ein oder mehrere Nickelsalze, eine oder mehrere anorganische Säuren und ein oder mehrere Glanzmittel enthalten, dadurch gekennzeichnet, daß man als Glanzmittel Thiuroniumsalze der allgemeinen Formel I 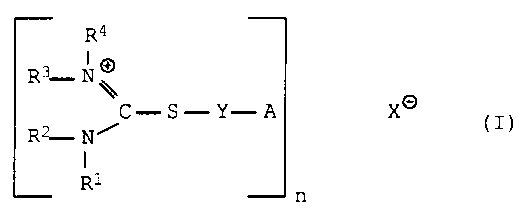 in der
R¹ bis R⁴ Wasserstoff, C₁- bis C₁₈-Alkyl, welches durch Carboxylgruppen, C₁- bis C₄-Alkoxycarbonylgruppen oder Cyanogruppen substituiert sein kann, C₂- bis C₁₂-Alkenyl, C₂- bis C₁₂-Alkinyl, C₅- bis C₈-Cycloalkyl, C₇- bis C₁₂-Phenylalkyl oder Phenyl, welches durch ein oder zwei C₁- bis C₄-Alkylreste, C₁- bis C₄-Alkoxyreste, Halogenatome, Hydroxylgruppen, Phenylreste oder C₁- bis C₄-Alkoxycarbonylgruppen substituiert sein kann, bedeuten,
Y eine chemische Bindung oder lineares oder verzweigtes Alkylen, Alkenylen oder Alkinylen mit jeweils bis zu 20 C-Atomen bezeichnet,
A Wasserstoff oder eine Gruppierung der Formel -CO-H, -CO-R⁵, -CO-OH, -CO-OR⁵, -CO-NR⁶R⁷, -CO-CH₂-CO-OR⁵, -O-CO-H, -O-CO-R⁵, -NR⁶-CO-R⁵, -OR⁵, -SO₂-R⁵, -SO₂-OH, -SO₂-OR⁵, -PO(OH)₂, -PO(OH)(OR⁵), -PO(OR⁵)₂, OPO(OH)₂, -OPO(OH) (OR⁵) oder -OPO(OR⁵)₂ bezeichnet, wobei
R⁵ C₁- bis C₁₂-Alkyl, C₂- bis C₁₂-Alkenyl, C₂- bis C₁₂-Alkinyl, C₅- bis C₈-Cycloalkyl, C₇- bis C₁₂-Phenylalkyl oder Phenyl, welches durch ein oder zwei C₁- bis C₄-Alkylreste, Halogenatome, Hydroxylgruppen, Phenylreste oder C₁- bis C₄-Alkoxycarbonylgruppen substituiert sein kann, bedeutet und
R⁶ und R⁷ Wasserstoff oder C₁- bis C₄-Alkyl bedeuten,
n für eine Zahl von 1 bis 4 steht und
X^{⊖} ein n-wertiges anorganisches oder organisches Anion, welches die Wasserlöslichkeit fördert, bezeichnet,
einsetzt.

2. Verfahren zur Herstellung vernickelter Formteile nach Anspruch 1, dadurch gekennzeichnet, daß man als Glanzmittel Thiuroniumsalze I einsetzt, bei denen die Reste R¹ bis R⁴ Wasserstoff, C₁- bis C₄-Alkyl, Allyl oder Phenyl, welches durch ein oder zwei C₁- bis C₄-Alkylreste substituiert sein kann, bedeuten.

3. Verfahren zur Herstellung vernickelter Formteile nach Anspruch 1 oder 2, dadurch gekennzeichnet, daß man als Glanzmittel Thiuroniumsalze der allgemeinen Formel Ia 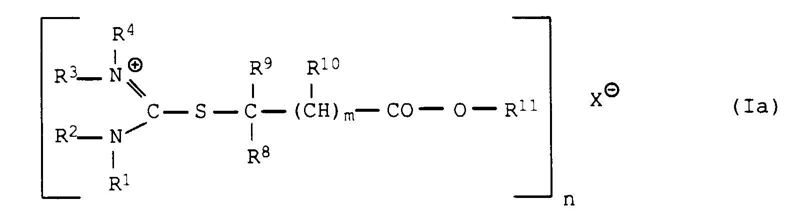 einsetzt, in der die Variablen R¹ bis R⁴, n und X^{⊖} die oben genannten Bedeutungen haben, R⁸ bis R¹⁰ Wasserstoff oder C₁-bis C₄-Alkyl bezeichnen, R¹¹ Wasserstoff, C₁- bis C₁₂-Alkyl, C₂- bis C₁₂-Alkenyl, C₂- bis C₁₂-Alkinyl, C₅- bis C₈-Cycloalkyl, C₇- bis C₁₂-Phenylalkyl oder Phenyl, welches durch ein oder zwei C₁- bis C₄-Alkylreste substituiert sein kann, bedeutet und m für eine Zahl von 0 bis 10 steht.

4. Verfahren zur Herstellung vernickelter Formteile nach Anspruch 1 oder 2, dadurch gekennzeichnet, daß man als Glanzmittel Thiuroniumsalze der allgemeinen Formel Ib 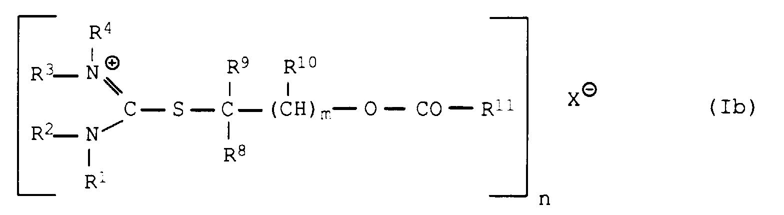 einsetzt, in der die Variablen R¹ bis R⁴, R⁸ bis R¹¹, m, n und X^{⊖} die oben genannten Bedeutungen haben.

5. Verfahren zur Herstellung vernickelter Formteile nach Anspruch 1 oder 2, dadurch gekennzeichnet, daß man als Glanzmittel Thiuroniumsalze der allgemeinen Formel Ic 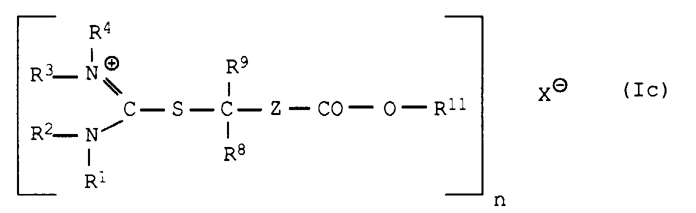 einsetzt, in der die Variable R¹ bis R⁴, R⁸, R⁹, R¹¹, n und X^{⊖} die oben genannten Bedeutungen haben und Z für eine Gruppierung der Formel -CR¹²=CR¹³ - oder -C≡C- steht, wobei R¹² und R¹³ Wasserstoff oder C₁- bis C₄-Alkyl bezeichnen.

6. Verfahren zur Herstellung vernickelter Formteile nach Anspruch 1 oder 2, dadurch gekennzeichnet, daß man als Glanzmittel Thiuroniumsalze der allgemeinen Formel Id 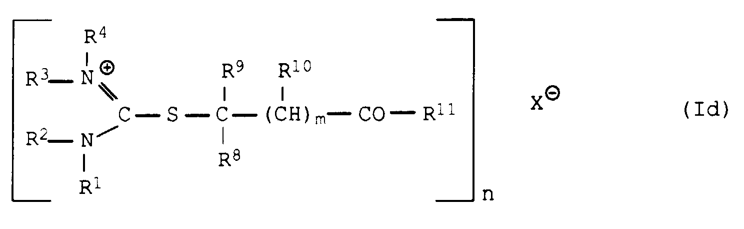 einsetzt, in der die Variablen R¹ bis R⁴, R⁸ bis R¹¹, m, n und X^{⊖} die oben genannten Bedeutungen haben.

7. Verfahren zur Herstellung vernickelter Formteile nach Anspruch 1 oder 2, dadurch gekennzeichnet, daß man als Glanzmittel Thiuroniumsalze der allgemeinen Formel Ie 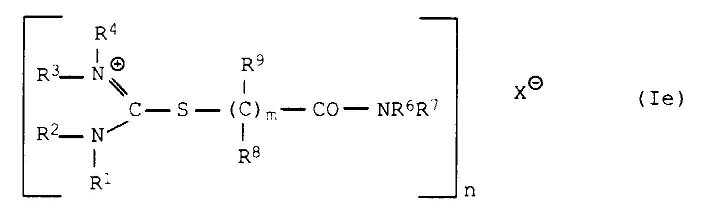 einsetzt, in der die Variablen R¹ bis R⁴, R⁶ bis R⁹, m, n und X^{⊖} die oben genannten Bedeutungen haben.

8. Verfahren zur Herstellung vernickelter Formteile nach Anspruch 1 oder 2, dadurch gekennzeichnet, daß man als Glanzmittel Thiuroniumsalze der allgemeinen Formel If 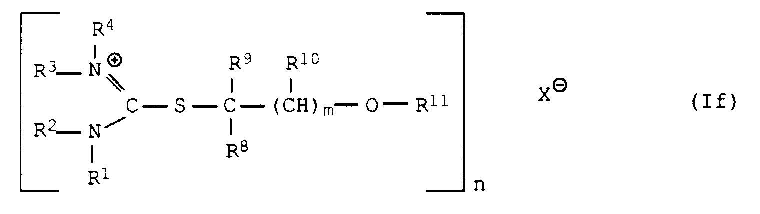 einsetzt, in der die Variablen R¹ bis R⁴, R⁸ bis R¹¹, m, n und X^{⊖} die oben genannten Bedeutungen haben.

9. Verfahren zur Herstellung vernickelter Formteile nach Anspruch 1 oder 2, dadurch gekennzeichnet, daß man als Glanzmittel Thiuroniumsalze der allgemeinen Formel Ig 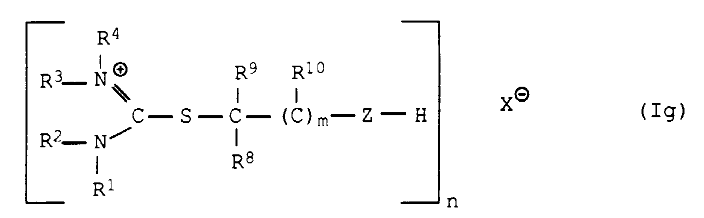 einsetzt, in der die Variablen R¹ bis R⁴, R⁸, R⁹, Z, m, n und X^{⊖} die oben genannten Bedeutungen haben.

10. Verfahren zur Herstellung vernickelter Formteile nach den Ansprüchen 1 bis 9, dadurch gekennzeichnet, daß man neben den Thiuroniumsalzen I mindestens ein weiteres Glanzmittel verwendet.

11. Thiuroniumsalze der allgemeinen Formel Ih 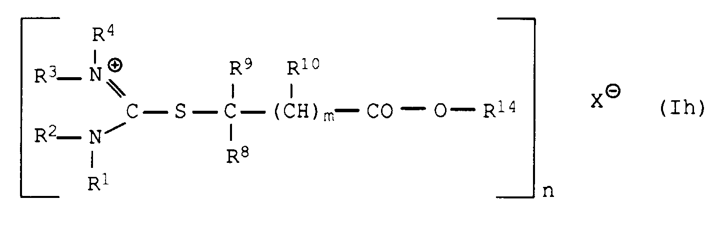 in der die Variablen R¹ bis R⁴, R⁸ bis R¹⁰, m, n und X^{⊖} die in Anspruch 1 oder 3 genannten Bedeutungen haben und R¹⁴ für C₇bis C₁₂-Phenylalkyl oder Phenyl, welches durch ein oder zwei C₁- bis C₄-Alkylreste substituiert sein kann, steht.

12. Thiuroniumsalze der allgemeinen Formel Ij 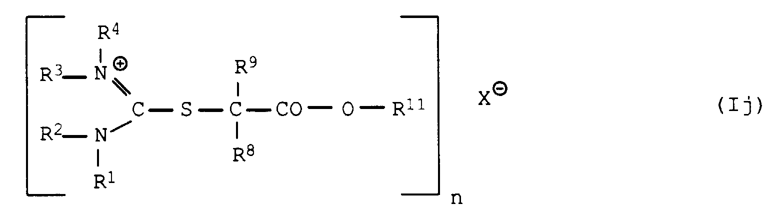 in der die Variablen R¹ bis R⁴, R⁸, R⁹, R¹¹, n und X^{⊖} die in Anspruch 1 oder 3 genannten Bedeutungen haben mit der Maßgabe, daß mindestens einer der Reste R⁸ und R⁹ von Wasserstoff verschieden ist.

13. Thiuroniumsalze der allgemeinen Formel Ib gemäß Anspruch 4 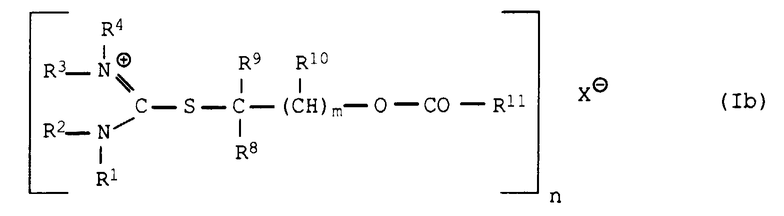 in der die Variablen R¹ bis R⁴, R⁸ bis R¹¹, m, n und X^{⊖} die oben genannten Bedeutungen haben.

14. Thiuroniumsalze der allgemeinen Formel Ik 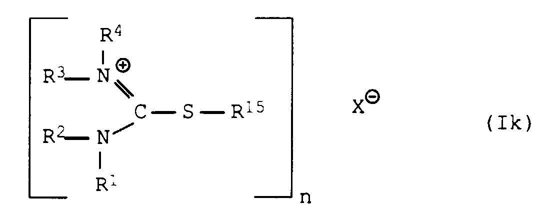 in der die Variablen R¹ bis R⁴, n und X^{⊖} die in Anspruch 1 genannten Bedeutungen haben und R¹⁵ für die Gruppierung 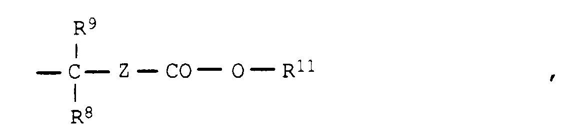 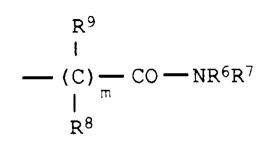 oder 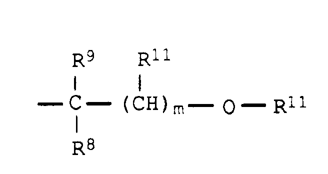 steht, wobei die Variablen R⁶ bis R¹¹, Z und m die in Anspruch 1, 3 oder 5 genannten Bedeutungen haben, mit der Maßgabe, daß mindestens einer der Reste R¹ bis R⁴ eine Allylgruppe bezeichnet.

## Claims

1. A process for producing nickelized shaped articles by electrodeposition of nickel from aqueous acidic baths including as essential constituents one or more nickel salts, one or more inorganic acids and one or more brighteners, which comprises using brighteners comprising thiouronium salts of the general formula I 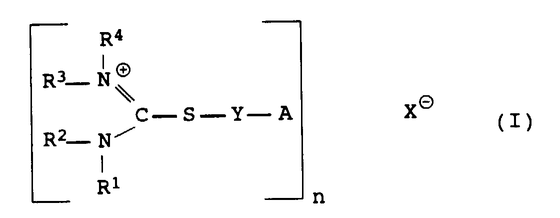 where
R¹ to R⁴ are each hydrogen, C₁-₁₈-alkyl, which may be carboxyl-, C₁-C₄-alkoxycarbonyl- or cyano-substituted, C₂-₁₂-alkenyl, C₂-C₁₂-alkynyl, C₅-C₈-cycloalkyl, C₇-C₁₂-phenylalkyl or phenyl which may be substituted by one or two substituents selected from the group consisting of C₁-C₄-alkyl, C₁-C₄-alkoxy, halogen, hydroxyl, phenyl and C₁-C₄-alkoxycarbonyl,
Y is a chemical bond or linear or branched alkylene, alkenylene or alkynylene having in each case up to 20 carbon atoms,
A is hydrogen or a group of the formula -CO-H, -CO-R⁵, -CO-OH, -CO-OR⁵, -CO-NR⁶R⁷, -CO-CH₂-CO-OR⁵, -O-CO-H, -O-CO-R⁵, -NR⁶-CO-R⁵, -OR⁵, -SO₂-R⁵, -SO₂-H, -SO₂-R⁵, -PO(OH)₂, -PO(OH)(OR⁵), -PO(OR⁵)₂, OPO(OH)₂, -OPO(OH)(OR⁵) or -OPO(OR⁵)₂, where
R⁵ is C₁-C₁₂-alkyl, C₂-C₁₂-alkenyl, C₂-C₁₂-alkynyl, C₅-C₈-cycloalkyl, C₇-C₁₂-phenylalkyl or phenyl which may be substituted by one or two substituents selected from the group consisting of C₁-C₄-alkyl, halogen, hydroxyl, phenyl and C₁-C₄-alkoxycarbonyl, and
R⁶ and R⁷ are each hydrogen or C₁-C₄-alkyl,
n is from 1 to 4, and
X^{⊖} is an n-valent inorganic or organic anion that promotes solubility in water.

2. A process as claimed in claim 1 wherein R¹ to R⁴ are each hydrogen, C₁-C₄-alkyl, allyl or phenyl which may be substituted by one or two C₁-C₄-alkyl radicals.

3. A process as claimed in claim 1 or 2 wherein the thiouronium salts used as brighteners have the general formula Ia 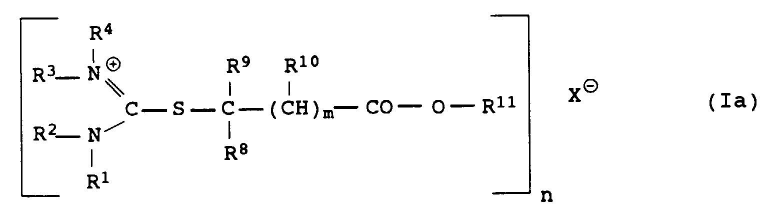 where R¹ to R⁴, n and X^{⊖} are each as defined above, R⁸ to R¹⁰ are each hydrogen or C₁-C₄-alkyl, R¹¹ is hydrogen, C₁-C₁₂-alkyl, C₂-C₁₂-alkenyl, C₂-C₁₂-alkynyl, C₅-C₈-cycloalkyl, C₇-C₁₂-phenylalkyl or phenyl which may be substituted by one or two C₁-C₄-alkyl radicals, and m is from 0 to 10.

4. A process as claimed in claim 1 or 2 wherein the thiouronium salts used as brighteners have the general formula Ib 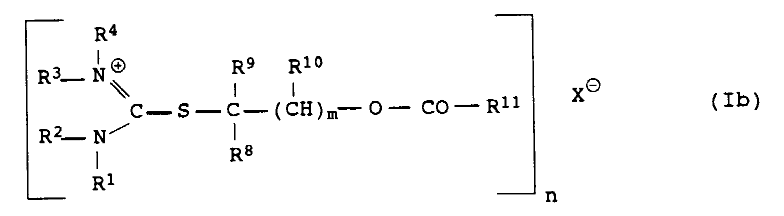 where R¹ to R⁴, R⁸ to R¹¹, m, n and X^{⊖} are each as defined above.

5. A process as claimed in claim 1 or 2 wherein the thiouronium salts used as brighteners have the general formula Ic 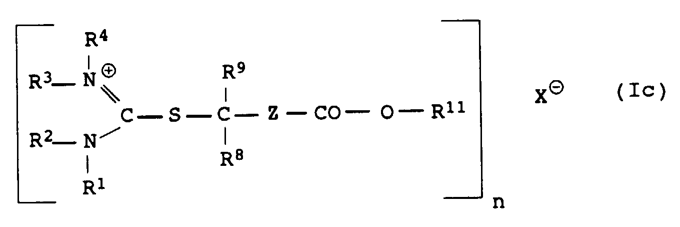 where R¹ to R⁴, R⁸, R⁹, R¹¹, n and X^{⊖} are each as defined above and Z is a group of the formula -CR¹²=CR¹³- or -C≡C- where R¹² and R¹³ are each hydrogen or C₁-C₄-alkyl.

6. A process as claimed in claim 1 or 2 wherein the thiouronium salts used as brighteners have the general formula Id 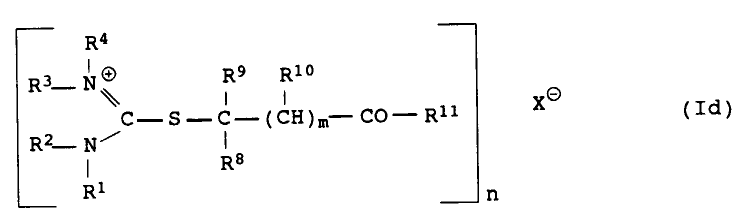 where R¹ to R⁴, R⁸ to R¹¹, m, n and X^{⊖} are each as defined above.

7. A process as claimed in claim 1 or 2 wherein the thiouronium salts used as brighteners have the general formula Ie 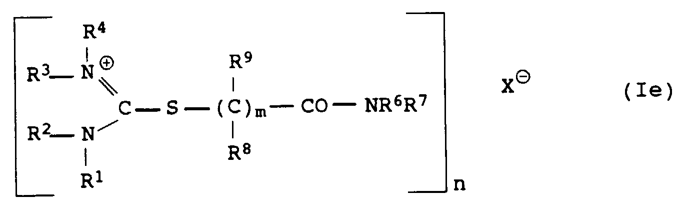 where R¹ to R⁴, R⁶ to R⁹, m, n and X^{⊖} are each as defined above.

8. A process as claimed in claim 1 or 2 wherein the thiouronium salts used as brighteners have the general formula If 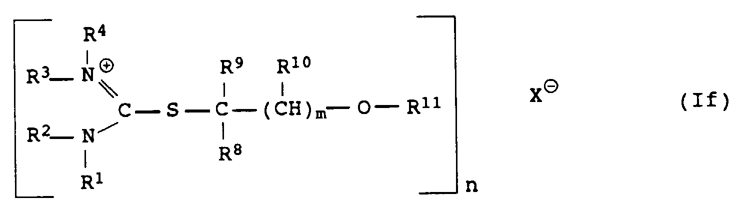 where R¹ to R⁴, R⁸ to R¹¹, m, n and X^{⊖} are each as defined above.

9. A process as claimed in claim 1 or 2 wherein the thiouronium salts used as brighteners have the general formula Ig 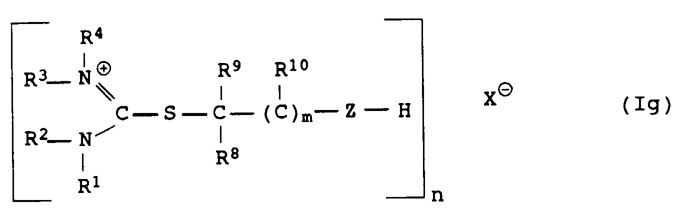 where R¹ to R⁴, R⁸, R⁹, Z, m, n and X^{⊖} are each as defined above.

10. A process as claimed in any of claims 1 to 9 wherein as well as the thiouronium salts I at least one further brightener is used.

11. Thiouronium salts conforming to the general formula Ih 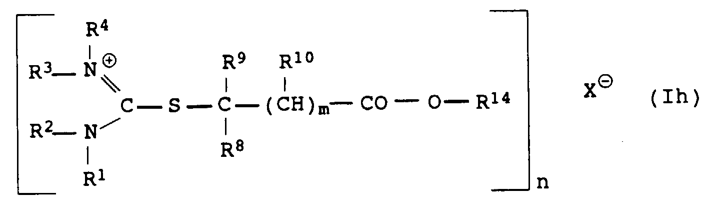 where R¹ to R⁴, R⁸ to R¹⁰, m, n and X^{⊖} are each as defined in claim 1 or 3 and R¹⁴ is C₇-C₁₂-phenylalkyl or phenyl which may be substituted by one or two C₁-C₄-alkyl radicals.

12. Thiouronium salts conforming to the general formula Ij 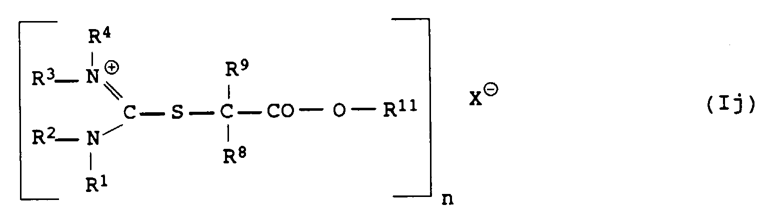 where R¹ to R⁴, R⁸, R⁹, R¹¹, n and X^{⊖} are each as defined in claim 1 or 3, subject to the proviso that at least one of R⁸ and R⁹ is not hydrogen.

13. Thiouronium salts of the general formula Ib as set forth in claim 4 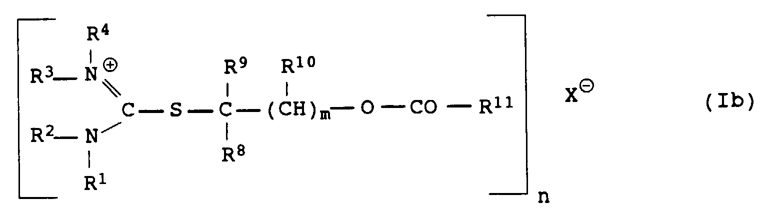 where R¹ to R⁴, R⁸ to R¹¹, m, n and X^{⊖} are each as defined above.

14. Thiouronium salts conforming to the general formula Ik 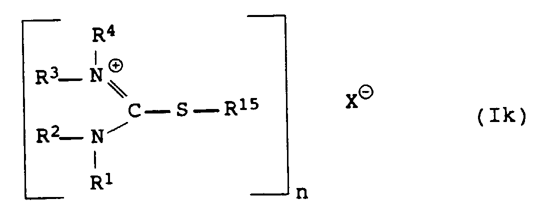 where R¹ to R⁴, n and X^{⊖} are each as defined in claim 1 and R¹⁵ is the group 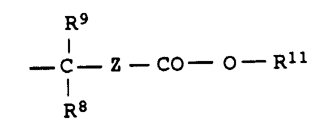 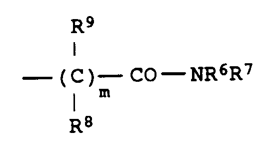 or 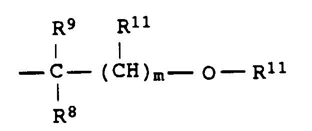 where R⁶ to R¹¹, Z and m are each as defined in claim 1, 3 or 5, subject to the proviso that at least one of R¹ to R⁴ is allyl.

## Revendications

1. Procédé de fabrication de pièces moulées nickelées par dépôt galvanique de nickel à partir de bains acides-aqueux qui contiennent, comme constituants essentiels, un ou plusieurs sels de nickel, un ou plusieurs acides inorganiques et un ou plusieurs brillanteurs, caractérisé en ce que l'on utilise, comme brillanteur, des sels de thio-uronium de formule générale I 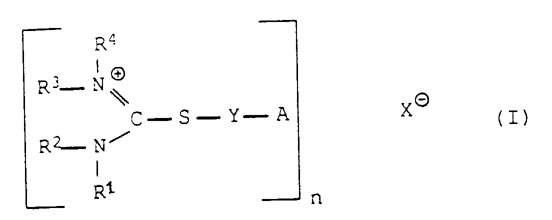 dans laquelle
R¹ à R⁴ représentent des atomes d'hydrogène, des restes alkyle en C₁-C₁₈ qui peuvent être substituée par des groupements carboxyle, des groupements (alcoxy en C₁-C₄)carbonyle ou des groupements cyano, des restes alcényle en C₂-C₁₂, alcynyle en C₂-C₁₂, cycloalkyle en C₅-C₈, phénylalkyle en C₇-C₁₂ ou phényle qui peut être substitué par un ou deux restes alkyle en C₁-C₄, alcoxy en C₁-C₄, atomes d'halogène, groupements hydroxy, restes phényle ou groupements (alcoxy en C₁-C₄)carbonyle,
Y représente une liaison chimique ou un reste alkyléne, alcénylène ou alcynylène linéaire ou ramifié, contenant chacun jusqu'à 20 atomes de carbone,
A représente un atome d'hydrogène ou un groupement de formule -CO-H, -COR⁵, -CO-OH, -CO-OR⁵, -CO-NR⁶R⁷, -CO-CH₂-CO-OR⁵, -O-CO-H, -O-CO-R⁵, -NR⁶-CO-R⁵, -OR⁵, -SO₂-R⁵, -SO₂-OH, -SO₂-OR⁵, -PO(OH)₂, -PO(OH)(OR⁵), -PO(OR⁵)₂, -OPO(OH)₂, -OPO(OH)(OR⁵) ou -OPO(OR⁵)₂, avec
R⁵: reste alkyle en C₁-C₁₂, alcényle en C₂-C₁₂, alcynyle en C₂-C₁₂, cycloalkyle en C₅-C₈, phénylalkyle en C₇-C₁₂ ou phényle qui peut être substitué par un ou deux restes alkyle en C₁-C₄, atomes d'halogène, groupements hydroxy, restes phényle ou groupements (alcoxy en C₁-C₄)carbonyle, et
R⁶ et R⁷: atomes d'hydrogène ou restes alkyle en C₁-C₄,
n est mis pour un nombre de 1 à 4 et
X⊖ représente un anion inorganique ou organique à n valences qui favorise la solubilité dans l'eau.

2. Procédé de fabrication de pièces moulées nickelées selon la revendication 1, caractérisé en ce que l'on utilise, comme brillanteur, des sels de thio-uronium I dans lesquels les restes R¹ à R⁴ représentent des atomes d'hydrogène ou des restes alkyle en C₁-C₄, allyle ou phényle qui peut être substitué par un ou deux restes alkyle en C₁-C₄.

3. Procédé de fabrication de pièces moulées nickelées selon la revendication 1 ou 2, caractérisé en ce que l'on utilise, comme brillanteur, des sels de thio-uronium de formule générale Ia 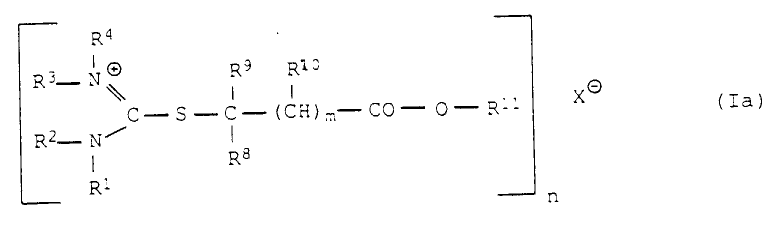 dans laquelle les variables R¹ à R⁴, n et X^{⊖} ont les significations données ci-dessus, R⁸ à R¹⁰ représentent des atomes d'hydrogène ou des restes alkyle en C₁-C₄, R¹¹ représente un atome d'hydrogène, un reste alkyle en C₁-C₁₂, alcényle en C₂-C₁₂, alcynyle en C₂-C₁₂, cycloalkyle en C₅-C₈, phénylalkyle en C₇-C₁₂ ou phényle qui peut être substitué par un ou deux restes alkyle en C₁-C₄, et m est mis pour un nombre de 0 à 10.

4. Procédé de fabrication de pièces moulées nickelées selon la revendication 1 ou 2, caractérisé en ce que l'on utilise, comme brillanteur, des sels de thio-uronium de formule générale Ib 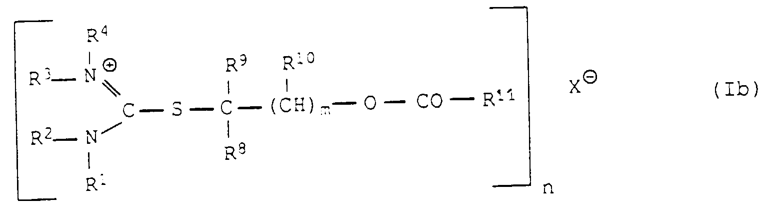 dans laquelle les variables R¹ à R⁴, R⁸ à R¹¹, m, n et X^{⊖} ont les significations données ci-dessus.

5. Procédé de fabrication de pièces moulées nickelées selon la revendication 1 ou 2, caractérisé en ce que l'on utilise, comme brillanteur, des sels de thio-uronium de formule générale Ic 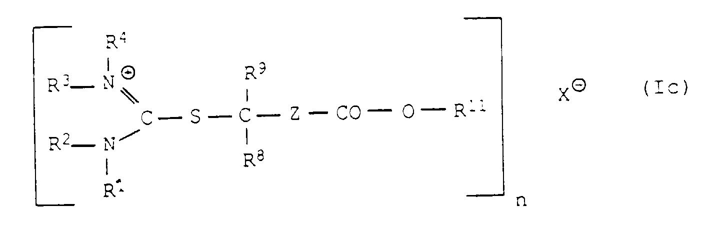 dans laquelle les variables R¹ à R⁴, R⁸, R⁹, R¹¹, n et X^{⊖} ont les significations données ci-dessus et Z est mis pour un groupement de formule -CR¹²=CR¹³- ou -C≡C-, R¹² et R¹³ représentant des atomes d'hydrogène ou des restes alkyle en C₁-C₄.

6. Procédé de fabrication de pièces moulées nickelées selon la revendication 1 ou 2, caractérisé en ce que l'on utilise, comme brillanteur, des sels de thio-uronium de formule générale Id 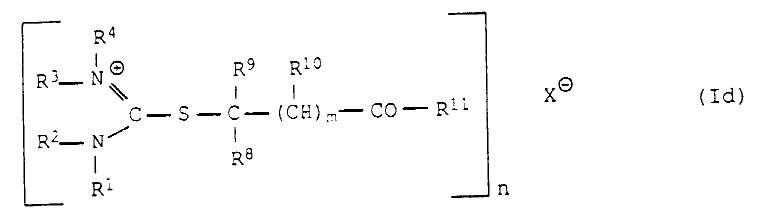 dans laquelle les variables R¹ à R⁴, R⁸ à R¹¹, m, n et X^{⊖} ont les significations données ci-dessus.

7. Procédé de fabrication de pièces moulées nickelées selon la revendication 1 ou 2, caractérisé en ce que l'on utilise, comme brillanteur, des sels de thio-uronium de formule générale Ie 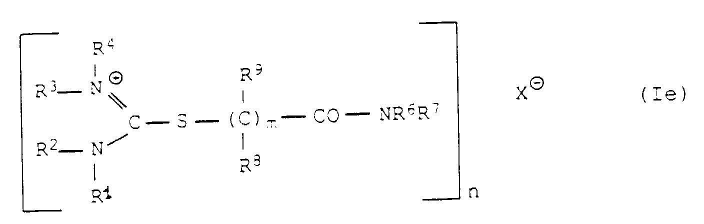 dans laquelle les variables R¹ à R⁴, R⁶ à R⁹, m, n et X^{⊖} ont les significations données ci-dessus.

8. Procédé de fabrication de pièces moulées nickelées selon la revendication 1 ou 2, caractérisé en ce que l'on utilise, comme brillanteur, des sels de thio-uronium de formule générale If 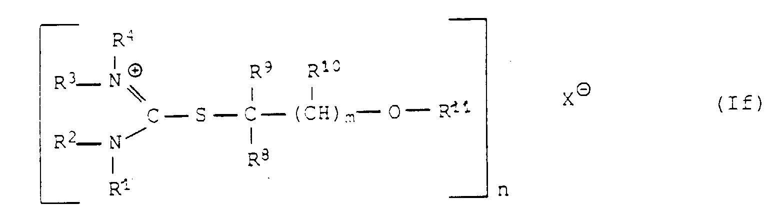 dans laquelle les variables R¹ à R⁴, R⁸ à R¹¹, m, n et X^{⊖} ont les significations données ci-dessus.

9. Procédé de fabrication de pièces moulées nickelées selon la revendication 1 ou 2, caractérisé en ce que l'on utilise, comme brillanteur, des sels de thio-uronium de formule générale Ig 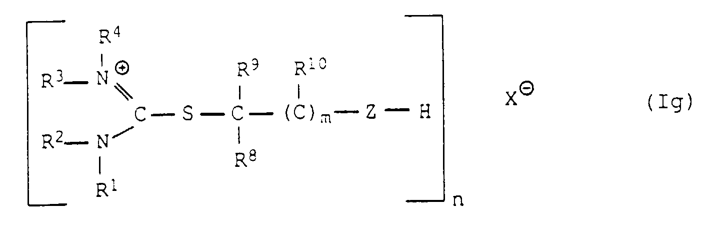 dans laquelle les variables R¹ à R⁴, R⁸, R⁹, Z, m, n et X^{⊖} ont les significations données ci-dessus.

10. Procédé de fabrication de pièces moulées nickelées selon l'une quelconque des revendications 1 à 9, caractérisé en ce que l'on utilise, en plus des sels de thio-uronium I, au moins un autre brillanteur.

11. Sels de thio-uronium de formule générale Ih 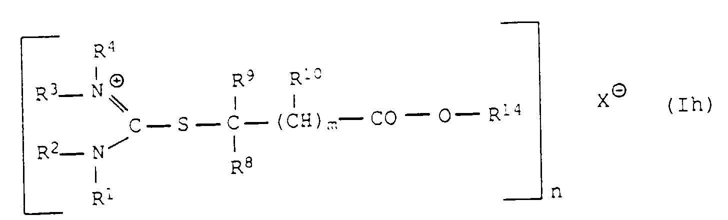 dans laquelle les variables R¹ à R⁴, R⁸ à R¹⁰, m, n et X^{⊖} ont les significations données dans la revendication 1 ou 3 et R¹⁴ est mis pour un reste phénylalkyle en C₇-C₁₂ ou un reste phényle qui peut être substitué par un ou deux restes alkyle en C₁-C₄.

12. Sels de thio-uronium de formule générale Ij 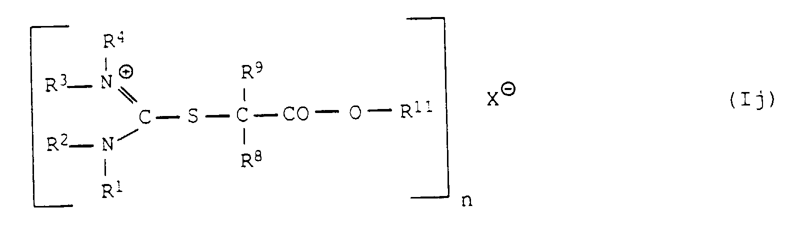 dans laquelle les variables R¹ à R⁴, R⁸, R⁹, R¹¹, n et X^{⊖} ont les significations données dans la revendication 1 ou 3, étant spécifié que l'un au moins des restes R⁸ et R⁹ est différent d'un atome d'hydrogène.

13. Sels de thio-uronium de formule générale Ib selon la revendication 4 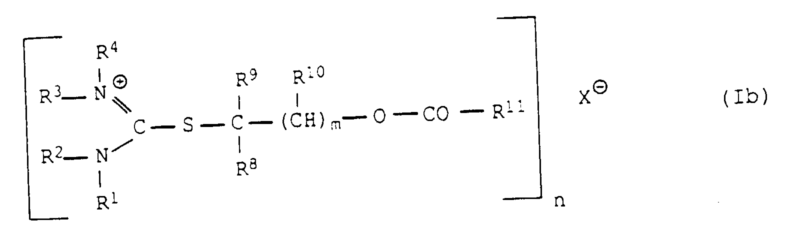 dans laquelle les variables R¹ à R⁴, R⁸ à R¹¹, m, n et X^{⊖} ont les significations données ci-dessus.

14. Sels de thio-uronium de formule générale Ik 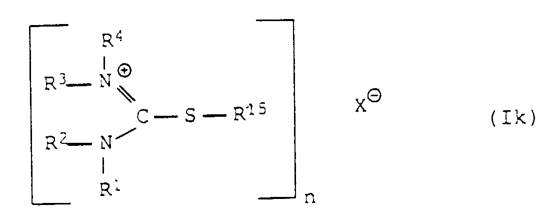 dans laquelle les variables R¹ à R⁴, n et X^{⊖} ont les significations données dans la revendication 1 et R¹⁵ est mis pour le groupement 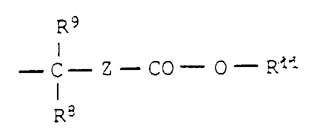 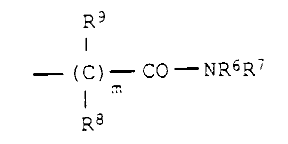 ou 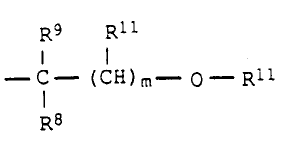 les variables R⁶ à R¹¹, Z et m ayant les significations données dans la revendication 1, 3 ou 5, étant spécifié que l'un au moins des restes R¹ à R⁴ représente un groupement allyle.
